# EUROPÄISCHE PATENTSCHRIFT

(11) **EP 1 292 278 B1**
(45) Veröffentlichungstag und Bekanntmachung des Hinweises auf die Patenterteilung: **05.12.2007**
(21) Anmeldenummer: 01962731.4
(22) Anmeldetag: 12.06.2001
(51) Int. Cl.: A61K 8/97, A61Q 19/08, A61Q 5/00

(54) **ZUBEREITUNGEN ENTHALTEND EINEN EXTRAKT DER PFLANZE PISTIA STRATIOTES**
PREPARATIONS THAT CONTAIN AN EXTRACT OF THE PLANT PISTIA STRATIOTES
PREPARATIONS RENFERMANT UN EXTRAIT DE PLANTES PISTIA STRATIOTES

(30) Priorität: 21.06.2000 FR 0007946
(43) Veröffentlichungstag der Anmeldung: 19.03.2003
(73) Patentinhaber: Cognis France, S.A.S., 31360 Boussens (FR)
(72) Erfinder: PAULY, Gilles, F-54000 Nancy (FR); DANOUX, Louis, F-54420 Saulxures les Nancy (FR); MOSER, Philippe, F-54270 Essey-les-Nancy (FR)
(74) Vertreter: Fabry, Bernd
(86) Internationale Anmeldenummer: PCT/EP2001/006606
(87) Internationale Veröffentlichungsnummer: WO 2001/097771

(56) Entgegenhaltungen:
- US-A- 5 773 397
- PATENT ABSTRACTS OF JAPAN vol. 1998, no. 10, 31. August 1998 (1998-08-31) & JP 10 139639 A (KAO CORP), 26. Mai 1998 (1998-05-26) in der Anmeldung erwähnt
- DATABASE WPI Section Ch, Week 199338 Derwent Publications Ltd., London, GB; Class D14, AN 1993-300792 XP002163373 & RO 105 006 A (INTR MECANICA UTIL CHIM BUCURESTI), 25. September 1992 (1992-09-25)
- PATENT ABSTRACTS OF JAPAN vol. 1996, no. 09, 30. September 1996 (1996-09-30) & JP 08 127511 A (MEIJI MILK PROD CO LTD), 21. Mai 1996 (1996-05-21)
- DATABASE BIOSIS [Online] BIOSCIENCES INFORMATION SERVICE, PHILADELPHIA, PA, US PREV199800367751, 1998 ACHOLA ET AL: 'Pharmacological activities of Pistia stratiotes'
- ALLIOTA G. ET AL: 'Potential allelochemicals from Pistia stratiotes L.' JOURNAL OF CHEMICAL ECOLOGY Bd. 17, Nr. 11, 1991, Seiten 2223 - 2234, XP001128735
- JANISTYN H.: 'HANDBUCH DER KOSMETIKA UND RIECHSTOFFE. III Band: Die Körperpflegemittel', 1973, DR. ALFRED HÜTHIG VERLAG, HEIDELBERG * Seite 666 - Seite 679 *

## Beschreibung

### Gebiet der Erfindung

Die Erfindung befindet sich auf dem Gebiet der Pflegestoffe und betrifft die Verwendung von Extrakten der Pflanze Pistia stratiotes in Kosmetik.

### Stand der Technik

Kosmetische Zubereitungen stehen dem Verbraucher heute in einer Vielzahl von Kombinationen zur Verfügung. Dabei wird nicht nur erwartet, dass diese Kosmetika einen bestimmten pflegenden Effekt zeigen oder einen bestimmten Mangel beheben, sondern immer häufiger wird nach Produkten verfangt, die mehrere Eigenschaften gleichzeitig aufweisen und somit ein verbessertes Leistungsspektrum zeigen. Von besonderem Interesse sind Stoffe, die sowohl Wirkstoffe darstellen, die für Haut und/oder Haare beispielsweise pflegende, vor Alterserscheinungen schützende, revitalisierende Eigenschaften vermitteln als auch gleichzeitig die technischen Eigenschaften des kosmetischen Produktes, wie Lagerstabilität, Lichtstabilität und Formulierbarkeit positiv beeinflussen oder zumindest nicht verschlechtern. Hierbei sind zusätzlich eine gute Hautverträglichkeit und besonders der Einsatz natürlicher Produkte beim Kunden gefragt. Daneben Ist es wünschenswert, durch Kombination bereits bekannter Wirkstoffe, oder durch auffinden neuer Einsatzgebiete bereits bekannter Substanzklassen deutlich bessere Produkte zu erhalten. Ein Nachteil besteht hier allerdings häufig darin, das eine Kombination von Wirkstoffen erst dann erhalten wird, wenn unterschiedliche Pflanzenextrakte gleichzeitig in unterschiedlichen Mengenverhältnissen verwendet werden.

Extrakte von Pflanzen und deren Inhaltstoffe finden immer häufiger Einsatz in der Kosmetik und Pharmazie. Pflanzenextrakte werden seit vielen Jahren in den unterschiedlichsten Kulturen für medizinische aber auch bereits für kosmetische Zwecke genutzt. Oftmals waren für diese Pflanzenextrakte nur ganz bestimmte einzelne Wirkungen bekannt und das Einsatzgebiet sehr eingeschränkt

### Beschreibung der Erfindung

Die Aufgabe der vorliegenden Patentanmeldung hat darin bestanden, Pflanzenextrakte aus einer Pflanze bereitzustellen, die einen Einsatz in der Kosmetik ermöglichen und neben pflegenden Eigenschaften vor allem vorbeugende und heilende Wirkung bei Alterserscheinungen der Haut zeigen, reaktivierend und revitalisierend wirken können und gleichzeitig als schlankmachende Mittel gegen Cellulitis einsetzbar sind.

Eine weitere Aufgabe der vorliegenden Patentanmeldung hat darin bestanden, Zubereitungen zur Verfügung zu stellen, die Wirkstoffe aus nachwachsenden Rohstoffen enthalten und gleichzeitig vielseitig als Pflegemittel In der Kosmetik sowohl in der Hautkosmetik, als auch in der Haarpflege einsetzbar sind.

Ein Gegenstand der Erfindung ist die Verwendung von Extrakten der Pflanze Pistia stratiotes in Pflegemitteln für die Haut und/oder die Haare. Diese Art der Verwendung umfasst Mittel mit kosmetischer Wirkung.

Überraschenderweise wurde gefunden, dass durch den Einsatz von Extrakten aus Pistia stratiotes Produkte erhalten werden, die gleichzeitig gute pflegende und schützende Eigenschaften für Haut und Haar aufweisen, sowie eine hohe Hautverträglichkeit besitzen. Die so erhaltenen Mittel zeichnen sich durch besonders gute Effekte in der Hautkosmetik aus. Sie zeigen neben schlankmachenden Eigenschaften und einem Anti-Cellulitis Effekt auch eine vorbeugende und hellende Wirkung bei Alterserscheinungen der Haut und eine revitalisierenden und reaktivierende Aktivität auf Haut und Haare.

Diese mehrfachen Einsatzgebiete der erfindungsgemäß zu verwendenden Mittel aus dem nachwachsendem Rohstoff der Pflanze Pistia stratiotes macht es für den Markt und für den Verbraucher sehr attraktiv. Die komplexe Aufgabe der Erfindung konnte somit durch den Einsatz eines Extraktes der Pflanze Pista stratiotes gelöst werden.

Der Begriff Zubereitungen wird im Rahmen der Erfindung synonym mit dem Begriff Mittel verwendet

Unter dem Begriff Pflanze im Sinne der vorliegenden Anmeldung sind sowohl ganze Pflanzen ats auch Pflanzenteile (Blätter, Wurzeln, Blüten) sowie deren Gemische zu verstehen.

### Pistia stratiotes

Die erfindungsgemäß einzusetzenden Extrakte werden aus Pflanzen der Familie Araceae, oder auch Familie der Aronstabgewächse gewonnen und zwar handelt es sich um Extrakte der Pflanze Pistia stratiotes. Bei dieser Pflanze handelt sich um eine sich weit ausbreitende, schwimmende Rosettenpflanze mit Wurzeln, die auch "Wassersalat" genannt wird. Sie ist in den Tropen und

Subtropen weit verbreitet. Der von einer flaschenförmigen Scheide umschlossene Kolben trägt unten eine weibliche und oben, von einer Art Manschette getrennt, eine männliche Blüte, die aus zwei zu einem Synandrium vereinten Staubblättern besteht Die Blütenstände sind so unscheinbar, dass sie von den breit-eiförmigen Blältern fast verdeckt werden (vergl. auch: Herder, Lexikon der Biologie, 6. Band, Spektrum Akademischer Verlag, Heidelberg, Berlin, Oxford).

In der traditionellen Medizin hat diese Pflanze bereits Anwendung gefunden zur Therapie der Dysuria und als Diuretikum. Die Wurzein wurden angewendet gegen Entzündungen und Verbrennungen. In einer Mischung mit Reis und Kokosnussöl wurden sie bei Dysenterie verabreicht. In Verbindung mit Rosenwasser und Zucker wurden sie bei Asthma oder Husten zur Linderung gegeben. Die ganze Pflanze wurde lange Zeit in der chinesischen Volksmedizin gegen Geschwüre oder Ekzeme und gegen Syphilis eingesetzt.

Die japanische Schrift JP 10139639 beschreibt einen Inhibitor des Haarwachstums, der eine Kombination aus vielen unterschiedlichen Pflanzenextrakten enthält, worunter sich auch ein Extrakt der Pflanze Pistia stratiotes befindet Auf weichen der kombinierten Pflanzenextrakte oder auf welche Kombination der vorhandenen Pflanzenextrakte dieser Effekt beruht, ist nicht offenbart.

Achola et al. beschreibt die pharmakologischen Eigenschaften von Pistia stratiotes in International Journal of Pharmacognosy, vol. 35, Nr. 5, S. 329-333.

### Extraktion

Die Herstellung der erfindungsgemäß einzusetzenden Extrakte erfolgt durch übliche Methoden der Extraktion von Pflanzen bzw. Pflanzenteilen. Bezüglich der geeigneten herkömmlichen Extraktionsverfahren wie der Mazeration, der Remazeration, der Digestion, der Bewegungsmazeration, der Wirbelextraktion, Ultraschallextraktion, der Gegenstromextraktion, der Perkolation, der Reperkolation, der Evakolation (Extraktion unter vermindertem Druck), der Diakolation und Festflüssig-Extraktion unter kontinuierlichem Rückfluß, die in einem Soxhiet-Extraktor durchgeführt wird, die dem Fachmann geläufig und im Prinzip alle anwendbar sind, sei beispielhaft auf Hagers Handbuch der Pharmazeutischen Praxis, (5. Auflage, Bd. 2, S. 1026-1030, Springer Verlag, Berlin-Heidelberg-New-York 1991) verwiesen. Als Ausgangsmaterial können frische oder getrocknete Pflanzen oder Pflanzenteile eingesetzt werden, üblicherweise wird jedoch von, Pflanzen und/oder Pflanzenteilen ausgegangen, die vor der Extraktion mechanisch zerkleinert werden können. Hierbei eignen sich alle dem Fachmann bekannten Zerkleinerungsmethoden, als Beispiel sei die Zerkleinerung mit einem Klingen enthaltenen Gerät genannt.

Als Lösungsmittel für die Durchführung der Extraktionen können vorzugsweise organische Lösungsmittel, Wasser oder Gemische aus organischen Lösungsmitteln und Wasser, insbesondere niedermolekulare Alkohole, Ester, Ether, Ketone oder halogenhaltige Kohlenwasserstoffe mit mehr oder weniger hohen Wassergehalten (destilliert oder nicht destilliert) vorzugsweise wässrig, alkoholische Lösungen mit mehr oder weniger hohen Wassergehalten, verwendet werden. Besonders bevorzugt ist die Extraktion mit Wasser, Methanol, Ethanol, Propanol, Butanol und deren Isomere, Aceton, Propylenglycolen, Polyethylenglycolen Ethylacetat, Dichlormethan, Trichlormethan sowie Mischungen hieraus. Die Extraktion erfolgt in der Regel bei 20 bis 100 °C, bevorzugt bei 80 bis 100°C, insbesondere bei Siedetemperatur der Lösungsmittel oder Lösungsmittelgemische. In einer möglichen Ausführungsform erfolgt die Extraktion unter Inertgasatmosphäre zur Vermeidung der Oxidation der Inhaltsstoffe des Extraktes. Die Extraktionszeiten werden vom Fachmann in Abhängigkeit vom Ausgangsmaterial, dem Extraktionsverfahren, der Extraktionstemperatur, vom Verhältnis Lösungsmittel zu Rohstoff u.a. eingestellt. Nach der Extraktion können die erhaltenen Rohextrakte gegebenenfalls weiteren üblichen Schritten, wie beispielsweise Aufreinigung, Konzentration und/oder Entfärbung unterzogen werden. Falls wünschenswert, können die so hergestellten Extrakte beispielsweise einer selektiven Abtrennung einzelner unerwünschter Inhaltsstoffe, unterzogen werden. Die Extraktion kann bis zu jedem gewünschten Extraktionsgrad erfolgen, wird aber gewöhnlich bis zur Erschöpfung durchgeführt. Typische Ausbeuten (= Trockensubstanzmenge des Extraktes bezogen auf eingesetzte Rohstoffmenge) bei der Extraktion getrockneter Pflanzen oder getrockneter Pflanzenteile gegebenenfalls entfettet, liegen im Bereich von 1 bis 20, vorzugsweise 4 bis 16, insbesondere 8 bis 12 Gew.-%. Die vorliegende Erfindung umfasst die Erkenntnis, dass die Extraktionsbedingungen sowie die Ausbeuten der Endextrakte je nach gewünschtem Einsatzgebiet gewählt werden können. Falls gewünscht, können die Extrakte anschließend beispielsweise einer Sprüh- oder Gefriertrocknung unterworfen werden.

Die Einsatzmenge der Pflanzenextrakte in den genannten Zubereitungen richtet sich nach der Konzentration der einzelnen Inhaltstoffe und nach der Art der Anwendungen der Extrakte. Die Gesamtmenge des Pflanzenexiraktes, der in den erfindungsgemäßen Zubereitungen enthalten ist, beträgt in der Regel 0,01 bis 25 Gew.-%, vorzugsweise 0,03 bis 5 Gew.-%, insbesondere 0.03 bis 0,6 Gew.-% bezogen auf die Endzubereitung, mit der Maßgabe, dass sich die Mengenangaben mit Wasser und gegebenenfalls weiteren Hilfe- und Zusatzsboffen zu 100 Gew.-% addieren.

Der Gesamtanteil der Hilfs- und Zusatzstoffe kann 1 bis 50, vorzugsweise 5 bis 40 Gew.-% - bezogen auf die Endzubereitung der kosmetischen und/oder pharmazeutischen Zubereitungen - betragen. Die Herstellung der Zubereitungen kann durch übliche Katt - oder Heißprozesse erfolgen; vorzugsweise arbeitet man nach der Phaseninversionstemperatur-Methode.

Aktivsubstanz im Sinne der Erfindung bezieht sich auf den Anteil an Substanzen sowie Hilfs- und Zusatzstoffen, die in dem Mittel enthaltend sind, mit Ausnahme des zusätzlich hinzugefügten Wassers.

### Extrakte:

Die erfindungsgemäß zu verwendenden Extrakte der Pflanze Pistia stratiotes enthalten in der Regel Inhaltsstoffe aus der Gruppe bestehend aus Sterolen, Carotinoiden, Proteinen, Kohlenhydraten. Fetten, Vitaminen und/oder Mineralsalzen. Die Extrakte sind je nach gewähltem Ausgangsmaterial und nach gewählter Extraktionsmethode unterschiedlich zusammengesetzt.

Unter Sterolen sind im Sinne der Erfindung Steroide zu verstehen, die sich aus der Pflanze Pistia stratiotes Isolieren lassen. Im Besonderen handelt es sich um Steroide, die nur an C-3 eine HydroxyGruppe, also formal Alkohole darstellen. Zusätzlich besitzen die 27 bis 30 C-Atome enthaltenden Sterole im allgemeinen eine C=C-Doppelbindung in 5/6-Stellung, seltener auch/oder In 7/8, 8/9 und anderen Positionen (z. B. 22/23). Als besonders bevorzugte Sterole isoliert aus der Pflanze Pistia stratiotes gelten Sterole wie Stigmasterin, Stigmastearytstearate und/oder Stigmast-4,22-dien-3-on.

Im Sinne der vorliegenden Erfindung sind unter **Carotinoiden** solche zu verstehen, die sich aus der Pflanze Pistia stratiotes isolieren lassen. Im Besonderen handelt es sich um Stoffe, die chemisch betrachtet 11- bis 12-fach ungesättigte Tetraterpene mit einem Grundgerüst mit 9 konjugierten Doppelbindungen, 8 Methylverzweigungen (einschließlich der möglichen Ringstrukturen) und einer β-lonon-Rlngstruktur an einem Molekülende darstellen, während sie sich in der Struktur des anderen Endes des Moleküls unterscheiden. Typische Carotinoide sind beispielsweise β-Carotin bzw. Provitamin A, α-Carotin, Lutein, Cryptoxanthin, Zeaxanthin und Lycopin. Der Anteil an Carotinoide im Extrakt der Pflanze Pistia stratiotes liegt zwischen 300 und 400 mg/kg Trockengewicht des Extraktes, insbesondere zwischen 320 bis 360 mg pro kg Trockengewicht.

Unter Proteinen sind im Sinne der Erfindung solche zu verstehen, die sich aus der Pflanze Pista stratiotes isolieren lassen. Ihr Anteil am Trockengewicht des Extraktes liegt zwischen 15 und 25 mg/kg, insbesondere 20 bis 22 mg/kg. Proteine sind ein Bestandteil des Pflanzenplasmas und finden sich daher in allen Pflanzenteilen.

Im Sinne der Erfindung sind unter Kohlenhydrate solche zu verstehen, die sich aus der Pflanze Pistia stratiotes Isolieren lassen. Bevorzugt darunter zu verstehen sind Cellulose, Glucan, Inulin, Agar-Agar Carrageenan und Alginsäure.

Im Sinne der vorliegenden Erfindung sind unter Fetten solche zu verstehen, die sich aus der Pflanze Pistia stratiotes isolieren lassen. Es handelt sich hier um feste, halbfeste oder flüssige, mehr oder weniger viskose Triglyceride der Pflanze die chemisch im wesentlichen aus gemischten Glycerinestern höherer Fettsäuren mit gerader Anzahl von Kohlenstoff-Atomen bestehen. Eine bevorzugte Fettsäure ist die Palmitinsäure. Der Anteil an Fette in der Trockenmasse der Extrakte liegt zwischen 6 und 10 mg/kg, bevorzugt im Bereich 8 bis 9 mg/kg.

Im Sinne der vorliegenden Erfindung sind unter Vitamine solche zu verstehen, die sich aus der Pflanze Pistia stratiotes isolieren lassen. Im Besonderen handelt es sich neben Retinol und Dehydroretinol (Vitamin A1 und A2) hier bevorzugt um Ascorbinsäure (Vitamin C), α-Tocopherol (Vitamin E), Thiamin (Vitamin B1), Riboflavin (Vitamin B2), Pyridoxal (Vitamin B6), Folsäure (Vitamin B9), Niacin (Vitamin B3) sowie Pantothenat, die In unterschiedlichen Anteilen Isoliert werden.

Im Sinne der vorliegenden Erfindung enthalten die Extrakte der Pflanze Pistia stratiotes Mineralien in Form von Salzen der Alkali oder Erdalkalimetalle. Die vorwiegend auftretenden Metalle sind Natrium, Kalium oder Calcium. Die Alkali- oder Erdalkalimetalle treten in Form ihrer Salze, vorwiegend jedoch in

Form ihrer Halogenide, Oxide bzw. Hydroxide. Phosphate, Carbonate, Sulfate oder Nitrate auf.

### Pflegemittel:

Als Pflegemittel im Sinne der Erfindung sind Pflegemittel für Haut und Haar zu verstehen. Diese Pflegemittel schließen unter anderem reinigende und aufbauende Wirkung für Haut und Haare ein.

Die erfindungsgemäß verwendeten Zubereitungen zeigen darüber hinaus eine hervorragende hautpflegende Wirkung bei gleichzeitig hoher Hautverträglichkeit. Außerdem zeigen sie eine gute Stablität, Insbesondere gegenüber oxidativer Zersetzung der Produkte.

Die Applikation kann sowohl topisch als auch oral In Form von Tabletten, Dragees, Kapseln, Säfte, Lösungen und Granulate erfolgen.

Ein weiterer Gegenstand der Erfindung ist die kosmetische Verwendung von Extrakten aus Pistia stratiotes in schlankmachenden Mitteln für die Haut mit einer Anti-cellulitis Aktivität.

Die Cellulitis, die auch als Orangen- oder Apfelsinenhaut bezeichnet wird, entsteht in der Subcutis. Diese Hautschicht stellt ein lockeres Bindegewebe dar mit Einlagerungen von Fettzellengruppen (Fettläppchen). Ihrem Aufbau gemäß ist die Subcutis Verschiebe- und Verbindungsschicht zwischen Haut und Unteriage, Nährstoff- und Wasserspeicher, Sitz der Druckrezeptoren. Ort des Fett- und Kohtenhydrat-Stoffwechsels und Durchgangsgebiet für größere Gefäße zur Hautoberfläche. Auf Grund der hohen Lipolyseaktivität werden die Extrakte erfindungsgemäß in schlankmachenden und Anti-Cellulites Mitteln eingesetzt. Die Lipolyseaktivität ist ein Maß für den körpereigenen Fettabbau in den Adipocyten

Ein weiterer Gegenstand der Erfindung ist die kosmetische Verwendung von Extrakten aus Pistia stratiotes in Pflegemitteln zur vorbeugenden oder heilenden Behandlung von Alterserscheinungen der Haut. Eine andere Bezeichnung für diese Art der Pflegemittel ist auch anti-ageing Mittel. Zu diesen Alterserscheinungen zählen beispielsweise jede Art der Fältchen- und Faltenbildung. Die Behandlungen schließen eine Verlangsamung von Altersprozessen der Haut mit ein. Die Alterserscheinungen können die unterschiedlichsten Ursachen aufweisen. Insbesondere sind diese Alterserscheinungen auf Grund einer durch UV-Strahlung induzierten Schädigung der Haut verursacht In einer besonderen Ausführungsform der Erfindung werden diese Pflegemittel zur Behandlung von durch UV-Strahlung induzierten Alterserscheinungen der Haut eingesetzt. In einer weiteren besonderen Ausführungsform der Erfindung werden diese Pflegemittel zur Behandlung von induzierter Apoptose und damit induzierten Aterserscheinungen der Haut eingesetzt, die durch einen Mangel an Wachstumsfaktoren hervorgerufen wurden.

Im Sinne der Erfindung versteht man unter Apoptose den gezielten Zelltod bestimmter unerwünschter oder geschädigter Zellen. Es handelt sich um einen aktiven Prozess der Zellen (Selbstmord auf Befehl). Apoptose wird durch einen oxidativen Stress (UV-Strahlung, Entzündung), durch einen Mangel an Wachstumsfaktoren oder durch giftige Stoffe (Schmutzstoffe, genotoxische Stoffe usw.) eingeleitet Bei der Hautalterung z. B. kann es durch einen Mangel an Wachstumsfaktoren in der Haut zu einer induzierten Apoptose der Hautzellen kommen. Bei den durch Apoptose betroffenen Zellen wird durch das spezifische Enzym Endonuclease die nukleare DNA abgebaut und die DNA-Fragmente in das Cytoplasma geschleust Als Wachstumsfaktoren im Sinne der Erfindung sind prinzipiell alle körpereigenen oder von außen zugeführten zu verstehen, die das Wachstum von Haut- und Haarzellen stimulieren. Dazu zählen beispielsweise Hormone und chemische Mediatoren oder Signalmoleküle. Es handelt sich zum Beispiel um Polypeptid-Wachstumsfaktoren oder Glykoprotein-Wachstumsfaktoren. Hier sei der Epidermale Wachstumsfaktor (EGF) genannt, der aus 53 Aminosäuren besteht und damit einen Polypeptid Wachstumsfaktor darstellt oder das Fibrillin, welches zu den Glykoproteinen gehört. Weitere Wachstumsfaktoren sind beispielsweise Urogastron, Laminin, Follistatin und Heregelin.

Ein weiterer Gegenstand der Erfindung ist die kosmetische Verwendung von Extrakten aus Pistia stratiotes in schützenden und aufbauenden Pflegemitteln mit revitalisierenden und reaktivierenden Aktivitäten für die Haut und/oder Haare. Diese Art der Verwendung dieser Pflegemittel, wirkt positiv beispielsweise gegen den negativen Einfluss der Umweltverschmutzung auf Haut und/oder Haare, indem sie die natürlichen Funktionen von Haut und/oder Haare reaktivieren und die Haut und/oder Haare widerstandsfähiger machen. Die revitalisierende und reaktivierende Aktivität von Extrakten der Pflanze Pistia stratiotes wirkt der Apoptosis entgegen. Die erfindungsgemäße Verwendung der Extrakte als schützende und aufbauende Pflegemittel ist prinzipiell für alle Zubereitungen möglich, die zur Prävention gegen Schädigungen oder bei Schädigungen der Haut und/oder Haare und damit In der Haut- und Haarpflege eingesetzt werden. Eine andere Verwendung auf diesem Gebiet ist die Applikation bei empfindlicher, durch Allergie oder anderen Ursachen geschädigter Haut. Die Schädigung der Haut kann dabei unterschiedlichste Ursachen haben.

Die Herstellung eines Extraktes der Pflanze Pistia stratiotes, kann nach einem Verfahren erfolgen, bei dem zur Extraktion als Extraktionsmedium Lösungsmittel oder Mischungen dieser

Lösungsmittel verwendet werden, welche ausgewählt sind aus der Gruppe, die gebildet wird von destilliertem oder nicht destilliertem Wasser, niedermolekularen Alkoholen, Estern, Ethern Kohlenwasserstoffe, Ketone oder halogenhaltige Kohlenwasserstoffe. Besonders bevorzugt als Extraktionsmittel ist destilliertes oder nicht destilliertes Wasser, niedermolekulare Alkohole wie Methanol, Ethanol, Propanol, Butanol und deren Isomere als reines Lösungsmittel oder als Lösungsmittel mit technischem Reinheitsgrad sowie als wässrige Lösungen, wobei der Gehalt an Wasser je nach Alkohol und Extraktionsmethode unterschiedlich sein kann. Die Extraktion erfolgt In der Regel bei 20 bis 100 °C, bevorzugt bei 80 bis 100°C, insbesondere bei Siedetemperatur der Lösungsmittel oder Lösungsmittelgemische.

Die erfindungsgemäßen zu verwendenden Extrakte können zur Herstellung von kosmetischen und/oder pharmazeutischen Zubereitungen, wie beispielsweise Haarshampoos, Haarlotionen, Schaumbäder, Duschbäder, Cremes, Gele, Lotionen, alkoholische und wäßrig/alkoholische Lösungen, Emulsionen, Wachs/Fett-Massen, Stiftpräparaten, Pudern oder Salben zum Einsatz kommen. Des weiteren können die erfindungsgemäßen zu verwendenden Extrakte zur oralen Applikation auch in Tabletten, Dragees, Kapseln, Säfte, Lösungen und Granulate eingearbeitet sein. Die orale Applikation ist besonders bevorzugt in Pflegemitteln mit schlankmachenden und Anti-cellulitis Eigenschaften für die Haut.

Diese Zubereitungen können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren,

Polymere, Siliconverbindungen, Fette, Wachse, Lecithine, Phospholipide, biogene Wirkstoffe. UV-Lichtschutzfaktoren, Antioxidantien. Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

### Tenside

Als oberflächenaktive Stoffe können anionische, nichtionische, kationische und/oder amphotere bzw. amphotere Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate. Alkansulfonate, Olefinsulfonate, Alkylethersulfonate, Glycerinethersulfonate, α-Methylestersulfonate, Sulfofettsäuren, Alkylsulfate. Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise J.Falbe (ed.), "Surfactants in Consumer Products", Springer Verlag, Berlin, 1987, S. 54-124 **oder** J.Falbe (ed.), "Katalysatoren, Tenside und Mineralöladditive", Thieme Verlag, Stuttgart, 1978, S. 123-217 verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, lsostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen (vgl. DE 19756377 A1), insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
➢ Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
➢ Alkyl- und/oder Alkenyloligoglykoside mit 8 bis 22 Kohlenstoffatomen im Alk(en)ylrest und deren ethoxylierte Analoga;
➢ Anlagerungsprodukte von 1 bis 15 Mol Ethylenoxid an Ricinusöl und/oder gehärtetes Ricinusöl;
➢ Anlagerungsprodukte von 15 bis 60 Mol Ethylenoxid an Ricinusöl und/oder gehärtetes Ricinusöl;
➢ Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Mischester aus Pentaerythrit, Fettsäuren, Citronensäure und Fettalkohol gemäß DE 1165574 PS und/oder Mischester von Fettsäuren mit 6 bis 22 Kohlenstoffatomen, Methylglucose und Polyolen, vorzugsweise Glycerin oder Polyglycerin.
➢ Mono-, Di- und Trialkylphosphate sowie Mono-, Di- und/oder Tri-PEG-alkylphosphate und deren Salze;
➢ Wollwachsalkohole;
➢ Polysiloxan-Polyalkyl-Polyether-Copolymere bzw. entsprechende Derivate;
➢ Block-Copolymere z.B. Polyethylenglycol-30 Dipolyhydroxystearate;
➢ Polymeremulgatoren, z.B. Pemulen-Typen (TR-1,TR-2) von Goodrich;
➢ Polyalkylenglycole sowie
➢ Glycerincarbonat.

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus DE 2024051 **PS** als Rückfettungsmittel für kosmetische Zubereitungen bekannt.

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® Gl 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispiels-weise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl* Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgener und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminium- und/oder Zinkstearat bzw. -ricinoleat eingesetzt werden.

### Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide, wie z.B. beschrieben in der FR 2252840 A sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, VinylpyrrolidonNinylacrylat-Copolymere, Vinylacetat/Butylmaleat/Isobomylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/Methylmeth-acrylat/tertButylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage. Weitere geeignete Polymere und Verdickungsmittel sind in **Cosm.Toil. 108, 95 (1993)** aufgeführt.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in Cosm.Toil. 91, 27 (1976**).**

### UV-Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
➢ 3-Benzylidencampher bzw. 3-Benzylidennorcampher und dessen Derivate, z.B. 3-(4-Methylbenzyliden)campher wie in der EP 0693471 B1 beschrieben;
➢ 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoe-säureamylester;
➢ Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-iso-propylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexyl-ester;
➢ Triazinderivate, wie z.B. 2,4,6-Trianilino-(p-carbo-2'-ethyl-1'-hexyloxy)-1,3,5-triazin und Octyl Triazon, wie in der EP 0818450 A1 beschrieben oder Dioctyl Butamido Triazone (Uvasorb® HEB);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der EP 0694521 B1 beschrieben.

Als wasserlösliche Substanzen kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ Sulfonsäurederivate von Benzophenonen, vorzugsweise 2-Hydroxy-4-methoxybenzo-phenon-5-sulfonsäure und ihre Salze;
➢ Sulfonsäurederivate des 3-Benzylidencamphers, wie z.B. 4-(2-Oxo-3-bornylidenmethyl)benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der DE 19712033 A1 (BASF). Die UV-A und UV-B-Filter können selbstverständlich auch in Mischungen eingesetzt werden. Besonders günstige Kombinationen bestehen aus den Derivate des Benzoylmethans" z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 543 (1996**)** sowie Parf.Kosm. 3, 11 (1999**)** zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Biogene Wirkstoffe

Unter biogenen Wirkstoffen sind im Rahmen der Erfindung zusätzlich solche zu verstehen, die nicht aus der Pflanze Pistia stratiotes stammen, wie beispielsweise Tocopherolacetat, Tocopherolpalmitat, Ascorbinsäure, (Desoxy)Ribonucleinsäure und deren Fragmentierungsprodukte, Retinol, Bisabolol, Allantoin, Phytantriol, Panthenol, AHA-Säuren, Aminosäuren, Ceramide, Pseudoceramide, essentielle Öle, weitere Pflanzenextrakte und zusätzliche Vitaminkomplexe.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren. Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
➢ adstringierende Wirkstoffe,
➢ Ölkomponenten,
➢ nichtionische Emulgatoren,
➢ Coemulgatoren,
➢ Konsistenzgeber,
➢ Hilfsstoffe wie z. B. Verdicker oder Komplexierungsmittel und/oder
➢ nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirko-nium-tetrachlorohydrat, Aluminium-Zirkonium-pentachlorohydrat und deren Komplexverbindungen z. B, mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
➢ entzündungshemmende, hautschützende oder wohlriechende ätherische Öle,
➢ synthetische hautschützende Wirkstoffe und/oder
➢ öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ytmethyl)-1,3-dioxylan-c-4-yimethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108, 95 (1993**)** entnommen werden.

### Insekten-Repellentien

Als Insekten-Repellentien kommen N,N-Diethyl-m-toluamid, 1,2-Pentandiol oder Ethyl Butylacetylaminopropionate in Frage

### Selbstbräuner und Depigimentierungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
➢ Glycerin;
➢ Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
➢ technische Oligoglyceringemische mit einem Eigenkondensationsgrad von 1,5 bis 10 wie etwa technische Diglyceringemische mit einem Diglyceringehalt von 40 bis 50 Gew.-%;
➢ Methyolverbindungen, wie insbesondere Trimethylolethan, Trimethylolpropan, Trimethylolbutan, Pentaerythrit und Dipentaerythrit;
➢ Niedrigalkylglucoside, insbesondere solche mit 1 bis 8 Kohlenstoffen im Alkylrest, wie beispielsweise Methyl- und Butylglucosid;
➢ Zuckeralkohole mit 5 bis 12 Kohlenstoffatomen, wie beispielsweise Sorbit oder Mannit,
➢ Zucker mit 5 bis 12 Kohlenstoffatomen, wie beispielsweise Glucose oder Saccharose;
➢ Aminozucker, wie beispielsweise Glucamin;
➢ Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen.

### Parfümöle

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

### Farbstoffe

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind. Diese Farbstoffe werden üblicherweise in Konzentrationen von 0,001 bis 0,1 Gew.-%, bezogen auf die gesamte Mischung, eingesetzt.

### Beispiele

### 1. Beispiel: Extraktion der Pflanzen mit destilliertem Wasser

300 g getrocknete Pistia stratiotes Pflanzen wurden in einer mit Klingen versehenen Zerkleinerrungsmaschine grob zerkleinert und dann in einen Glasreaktor mit 4,5 I destilliertem Wasser überführt. Der Aufguß wurde zwischen 80 und 85 °C erhitzt und unter Rühren über einen Zeitraum von 1 h bei dieser Temperatur extrahiert. Anschließend wurde die Mischung auf 20 °C abgekühlt und 15 min bei einer Geschwindigkeit von 5000 g zentrifugiert. Die überstehende kolloide Flüssigkeit wurde durch Filtration an Tiefenfilter mit einer mittleren Porosität von 450 nm (von der Firma Seitz, Bordeaux Frankreich) vom Rückstand getrennt. Der Extrakt wurde bei einer Anfangstemperatur von 185 °C und einer Endtemperatur von 80 °C sprühgetrocknet. Die Ausbeute an Trockenprodukt betrug 8,8 bis 12,1 Gew.-% bezogen auf das Trockengewicht an eingesetzten Pflanzen.

### 2. Beispiel: Extraktion der Pflanzen mit wässrigem Methanol

Beispiel 1 wurde wiederholt, die Extraktion jedoch mit 3 1 80 Gew.-%-igem wässrigen Methanol durchgeführt. Die Extraktion wurde unter Rühren 1 h bei Siedetemperatur unter reflux durchgeführt und der Extrakt wie beschrieben weiter verarbeitet. Die Filtration wurde wie im Beispiel 1 beschrieben durchgeführt. Anschließend wurde zunächst der Alkohol bei 45 °C unter vermindertem Druck entfernt und dann der grün-braune Rückstand wie beschrieben sprühgetrocknet. Die Ausbeute an Trockenprodukt betrug 7 bis 8 Gew.-% bezogen auf das Trockengewicht an eingesetzten Pflanzen.

### 3. Beispiel: Extraktion der Pflanzen mit wässrigem Ethanol

Beispiel 1 wurde wiederholt, die Extraktion jedoch mit 3 1 96 Gew.-%-igem wässrigen Ethanol durchgeführt. Die Extraktion wurde unter Rühren 1 h bei Siedetemperatur unter reflux durchgeführt und der Extrakt wie beschrieben weiter verarbeitet. Die Filtration wurde wie im Beispiel 1 beschrieben durchgeführt und der Rückstand nochmals mit 0,75 196 Gew.-%-igem wässrigen Ethanol gewaschen. Anschließend wurde zunächst der Alkohol bei 45 °C unter vermindertem Druck entfernt und dann der grüne Rückstand bei 50 °C getrocknet. Die Ausbeute an Trockenprodukt betrug 1,5 bis 5,3 Gew.-% bezogen auf das Trockengewicht an eingesetzten Pflanzen.

### 4. Beispiel: Inhibierung der Apoptose-Induktion

Hintergrund: Die Apoptose ist im Gegensatz zur Nekrose ein natürlicher gezielter Zelltod bestimmter unerwünschter oder geschädigter Zellen. Es handelt sich um einen aktiven Prozess der Zellen (Selbstmord auf Befehl). Apoptose kann durch einen oxidativen Stress (UV-Strahlung, Entzündung), durch einen Mangel an Wachstumsfaktoren oder durch giftige Stoffe (Schmutzstoffe, genotoxische Stoffe usw.) eingeleitet werden. Bei der Hautalterung z. B. kann es durch einen Mangel an Wachstumsfaktoren in der Haut zu einer induzierten Apoptose der Hautzellen kommen. Bei den durch Apoptose betroffenen Zellen wird durch das spezifische Enzym Endonuclease die nukleare DNA abgebaut und die DNA-Fragmente in das Cytoplasma geschleust.

Methode: Untersucht wurde die Fähigkeit der Pflanzenextrakte aus Pistia stratiotes, die durch einen Mangel an Wachstumsfaktoren induzierte Apoptose in humanen Hautzellen zu verhindern. An humanen Fibroblasten und humanen Keratinocyten wurden diese Test in vitro durchgeführt. Die humanen Zellen wurden in einem Nährmedium (DMEM = Dulbecco Minimum Essential Medium von der Firma Life Technologie Sarl) mit 10 % fötalem Kälberserum (von der Firma Dutcher) kultiviert. Diesem Nährmedium wurde Bromodesoxyuridin (BrdU) zugegeben, welches in die DNA eingebaut wurde und später zum Nachweis der DNA-Fragmente in dem Cytoplasma diente. Nach zwei Tagen Inkubationsdauer wurde das Nährmedium gegen Nährmedium (DMEM) ohne fötalem Kälberserum ausgetauscht.

Die zu testende Aktivsubstanz wurde zugegeben. Für den Pflanzenextrakt wurden zwei unterschiedliche Batch d.h. zwei unterschiedliche Extrakte (Batch A und B) der gleichen Extraktionsmethode getestet. Zum Vergleich wurde eine Zellprobe ohne zu testende Aktivsubstanz (Mengen und Konzentration angegeben in der Tabelle 1 und 2) inkubiert.

Nach einer Inkubationszeit von einem oder zwei Tagen bei 37 °C wurden die Zellen durch Trypsination zurückgewonnen nach der Methode von Dunnebacke und Zitcer beschrieben in: Cell and tissue culture, Hrsg.: J. Paul, Churchill Livingstone, 1975, S. 226. Nach der Trypsin-Behandlung wurden die Zellen zentrifugiert und ausgezählt. Anschließend wurde der Gehalt an BrdU in DNA-Fragmenten aus dem Cytoplasma mit Hilfe des ELISA Tests (ELISA Kit der Firma Roche) ermittelt. Der Gehalt an BrdU ist ein Maß für die DNA-Fragmente, die aus dem Nukleus, dem Zellkern, in das Cytoplasma geschleust wurden. Die Ergebnisse wurden auf eine Millionen Zellen bezogen und in Prozent im Vergleich zur Kontrolle angegeben.

**Tabelle 1: Anzahl der Zellen und Gehalt an DNA-Fragmenten im Cytoplasma nach der Behandlung von humanen Fibroblasten mit Extrakten von Pistia stratiotes.**

| **Zellzählung** | **Batch** | | **Gehalt an DNA-Fragmenten** | **Batch** | |
|---|---|---|---|---|---|
| | **A** | **B** | | **A** | **B** |
| Kontrolle | 100 | 100 | Kontrolle | 100 | 100 |
| Extrakt nach Beispiel 1; 0,01 Gew-% | 101 | 97 | Extrakt nach Beispiel 1 ; 0,01 Gew-% | 72 | 66 |
| Extrakt nach Beispiel 1; 0,03 Gew-% - | 97 | 101 | Extrakt nach Beispiel 1; 0,03 Gew-%- | 68 | 50 |
| | | | | | |

| **Zellzählung** | **Batch** | | **Gehalt an DNA-Fragmenten** | **Batch** | |
|---|---|---|---|---|---|
| | **A** | **B** | | **A** | **B** |
| Kontrolle | 100 | 100 | Kontrolle | 100 | 100 |
| Extrakt nach Beispiel 3; 0,01 Gew-%- | 104 | 106 | Extrakt nach Beispiel 3; 0,01 Gew-% | 74 | 63 |
| Extrakt nach Beispiel 3; 0,03 Gew-% | 124 | 112 | Extrakt nach Beispiel 3; 0,03 Gew-% | 46 | 50 |

**Tabelle 2: Anzahl der Zellen und Gehalt an DNA-Fragmenten im Cytoplasma nach der Behandlung von humanen Keratinocyten mit Extrakten von Pistia stratiotes**

| **Zellzählung** | **Batch** | | **Gehalt an DNA-Fragmenten** | **Batch** | |
|---|---|---|---|---|---|
| | **A** | **B** | | **A** | **B** |
| Kontrolle | 100 | 100 | Kontrolle | 100 | 100 |
| Extrakt nach Beispiel 1; 0,01 Gew-% | 92 | 92 | Extrakt nach Beispiel 1; 0,01 Gew-% | 58 | 64 |
| Extrakt nach Beispiel 1; 0,03 Gew-% | 89 | 92 | Extrakt nach Beispiel 1; 0,03 Gew-% | 44 | 61 |
| | | | | | |

| **Zellzählung** | **Batch** | | **Gehalt an DNA-Fragmenten** | **Batch** | |
|---|---|---|---|---|---|
| | **A** | **B** | | **A** | **B** |
| Kontrolle | 100 | 100 | Kontrolle | 100 | 100 |
| Extrakt nach Beispiel 3; 0,01 Gew-% | 94 | 109 | Extrakt nach Beispiel 3; 0,01 Gew-% | 87 | 64 |
| Extrakt nach Beispiel 3; 0,03 Gew-% | 93 | 111 | Extrakt nach Beispiel 3; 0,03 Gew-% | 73 | 60 |

Ergebnisse: Aus den Ergebnissen, dargestellt in den Tabellen 1 und 2, lässt sich erkennen, dass durch den Einsatz von Extrakten aus der Pflanze Pistia stratiotes die Apoptose in humanen Zellkulturen in vitro abnimmt. Der Gehalt an freien DNA-Fragmenten im Cytoplasma und damit der Grad an zerstörter DNA im Zellkern und der Grad an Apoptose nimmt mit zunehmender Konzentration an Pflanzenextrakt aus Pistia stratiotes ab. Die Werte der Zellzählung dokumentieren, dass die erfindungsgemäßen Pflanzenextrakte nicht toxisch sind und nicht zum Zelltod führen. Die Anzahl der vorhandenen intakten Zellen ist kaum verändert, der Gehalt an DNA-Fragmenten im Cytoplasma ist unter Einfluss des Pflanzenextraktes im Vergleich zur Kontrolle jedoch verringert. Der gezielte Zelltod wird durch diese Pflanzenextrakte unterdrückt. Diese Pflanzenextrakte zeigen einen "anti-ageing" Effekt an humanen Hautzellen.

### 5. Beispiel: Lipolyseaktivität an humanen Adipocyten

Hintergrund: Lipolyse ist die Bezeichnung für den körpereigenen Abbau von Fetten, die in den Adipocyten (Fettzellen) als Reserven vorhanden sind. Diese werden zu kleineren molekularen Bruchstücken, den Fettsäuren und dem Glycerin enzymatisch durch Lipasen gespalten. Die freien Fettsäuren werden dann von den Muskelzellen zur Energiegewinnung genutzt.

Methode: Die Adipocyten wurden aus humanem subkutanem Gewebe isoliert, wie es der allgemeinen Technik nach Rodbell (J. of Biolog. Chem.; 1964, 239, 375-380.) entspricht. Der Extrakt nach Beispiel 1 bis 3 mit je zwei Extrakte (Batch A und B) bzw. die Vergleichssubstanzen wurden im Referenzmedium einer definierten Salzkonzentration nach Hanks (aus: Cell and tissue culture Hrsg.: J. Paul, Churchill Livingstone, 1975, S. 484) gelöst und anschließend 90 Minuten bei 37 °C mit den isolierten Adipocyten in Kontakt gebracht. Es wurden je zwei bis sechs Adipocyten-Preparationen untersucht. Die prozentuale Erhöhung an freigesetztem Glycerin wurde spectrophotometrisch im Überstand des Mediums nach der Methode von Carpéné (J. de Pharmacologie; 1981; 12; 219-224.) bestimmt. Als Referenz wurde das Medium ohne zu untersuchende Substanz verabreicht (Referenzwert = 0).

**Tabelle 3: Lipolyseaktivität an humanen Adipocyten**

| **Substanz** | **Batch** | **Anzahl Präparationen** | **Konzentration Gew-%** | **%-Erhöhung an freigesetztem Glycerin** |
|---|---|---|---|---|
| Extrakt nach Beispiel 1 | A | 6 | 0,20 | 49 |
| | | 6 | 0,50 | 125 |
| | B | 6 | 0,20 | 36 |
| | | 6 | 0,50 | 108 |
| Extrakt nach Beispiel 2 | A | 4 | 0,02 | 35 |
| | | 4 | 0,05 | 23 |
| | | 4 | 0,10 | 51 |
| | B | 4 | 0,02 | 21 |
| | | 4 | 0,05 | 41 |
| | | 4 | 0,10 | 97 |
| Extrakt nach Beispiel 3 | A | 4 | 0,02 | 11 |
| | | 4 | 0,05 | 31 |
| | | 4 | 0,10 | 171 |
| | | 2 | 0,20 | 82 |
| | | 2 | 0,50 | 212 |
| | B | 4 | 0,10 | 13 |
| | | 4 | 0,20 | 32 |
| | | 4 | 0,60 | 95 |
| Theophylline (1,0 mM) | | 14 | | 187 |
| Isoprenalin (0,1 µM) | | 14 | | 129 |

Die Ergebnisse der Untersuchungen belegen, dass die Extrakte nach Beispiel 1 bis 3 aus Pistia stratiotes eine von der Konzentration abhängige, steigende Lipolyseaktivität bei humanen Adipocyten in vitro zeigen, da die prozentuale Erhöhung des freigesetzten Glycerins mit steigender Pflanzenextraktkonzentration ansteigt. Diese Ergebnisse belegen die Wirkung als schlankmachende Substanzen auf der Haut bzw. die anti-cellulitis Wirkung.

### 6. Regenerierende und revitalisierende Aktivität auf der Haut

Das Ziel dieser Test ist der Nachweis einer regenerierenden und revitalisierenden Aktivität von Extrakten aus Pistia stratiotes an humanen Fibroblastenkulturen in vitro.

Methode 1: Effekte am Zellwachstum Humane Fibroblasten wurden in einem definiertem Nährmedium (DMEM = Dulbecco Minimum Essential Medium, Firma Life Technologie Sarl) mit 10 Gew.-% fötalem Kälberserum angeimpft und für 24 h bei 37 °C in einer 5 %igen CO₂-Atmosphäre inkubiert. Anschließend wurde das Nährmedium mit fötalem Kälberserum durch ein Nährmedium aus DMEM ohne fötalem Kälberserum ausgetauscht. Zu diesem Nährmedium wurden unterschiedliche Konzentrationen an Aktivsubstanz in Form der Extrakte aus Pistia stratiotes nach Beispiel 1 bis 3 gegeben. Zum Vergleich wurde als Kontrolle eine Testreihe von humanen Fibroblasten ohne Aktivsubstanz inkubiert. Nach einer drei tägigen Inkubation der Fibroblasten im Nährmedium wurde das Wachstum und die Stoffwechselaktivität beurteilt, indem die Zellen mittels eines Partikelzählers ausgezählt wurden und indem der intrazelluläre Anteil an ATP nach der Methode von Vasseur (Journal francais Hydrologie, 1981, 9, 149-156.) bestimmt wurde. Bei Konzentrationen von 0,003 bis 0,01 Gew.-% Pflanzenextrakt nach Beispiel 1 bis 3 erhielt man eine Erhöhung des Anteils an ATP zwischen 16 und 43 % im Vergleich zur Kontrolle.

Die Studie zeigt, dass die Extrakte aus Pistia stratiotes nach Beispiel 1 bis 3 das Wachstum und den Stoffwechsel (Metabolismus) der humanen Fibroblasten in vitro erheblich anregt.

Methode 2: Verbesserung der Überlebensfähigkeit Die Test wurden durchgeführt an humanen Fibroblasten. Der Test ermöglicht auf den ruhenden Zellen eine gewisse Anzahl von Parametern mengenmäßig zu bestimmen. Die Kultivierung der Zellen entspricht der Kultivierung aus der Methode 1, ausgenommen der Inkubationszeit. Die Inkubationszeit für diese Test betrugen 72 h. Die Überlebensfähigkeit wurde beurteilt über die kolorimetrische Bestimmung des Anteils an Proteinen nach der Methode von Bradford (Anal. Biochem. 1976, 72, 248-254.) und über die Bestimmung des Anteils an Glutathion (GSH) mit einer fluoreszierenden Sonde, dem Orthophtalaldehyd nach der Methode von Hissin und Hilf (Anal. Biochem. 1976, 74, 214-216.). Das Glutathion wird von Zellen produziert um direkt gegen oxidativen Stress und Umwelteinflüssen wie hoher Schwermetallbelastung reagieren zu können. Ein erhöhter Anteil an reduziertem Glutathion nach Behandlung der Zellen mit Extrakten nach Beipsiel 1 bis 3, ist demnach ein Maß für die gesteigerte Überlebensfähigkeit der Zelle bei äußeren Stress und Belastungsbedingungen. Die Test wurden dreifach durchgeführt und dann zweimal wiederholt, so dass es sechs Ergebnisse pro Pflanzenextrakt und damit pro Batch gab, die jeweils gemittelt wurden. Die Ergebnisse wurden ermittelt in Prozent im Vergleich zur Kontrolle.

**Tabelle 4: Erhöhung des Proteingehaltes in humanen Fibroblasten nach Inkubation mit Extrakten nach Beispiel 1 bis 3 im Vergleich zur Inkubation ohne Pflanzenextrakt**

| **Substanz** | **Batch** | **Konzentration Gew-%** | **Gehalt an Proteine in%** |
|---|---|---|---|
| Kontrolle | | | 100 |
| Extrakt nach Beispiel 1 | A | 0,01 | 113 |
| | | 0,03 | 121 |
| | B | 0,01 | 139 |
| | | 0,03 | 143 |
| Extrakt nach Beispiel 2 | A | 0,01 | 119 |
| | | 0,03 | 133 |
| | B | 0,01 | 128 |
| | | 0,03 | 140 |
| Extrakt nach Beispiel 3 | A | 0,01 | 124 |
| | | 0,03 | 125 |
| | B | 0,01 | 128 |
| | | 0,03 | 149 |

Die Extrakte von Pistia stratiotes nach Beispiel 1 bis 3 in Konzentrationen von 0,01 bis 0,03 Gew.-% erhöhen den Gehalt an Proteinen in humanen Fibroblasten im Vergleich zur Kontrolle um 21 bis 49 % bei in vitro Untersuchungen. Die Erhöhung an Glutathion lag im Mittel bei 30 %. Diese Ergebnisse zeigen, dass die Extrakte von Pistia stratiotes eine hohe Kapazität zeigen um den Metabolismus von Fibroblasten anzuregen. Die Extrakte zeigen eine regenerierende und revitalisierende Aktivität an humanen Fibroblasten und sind damit als Energielieferanten und als anti-ageing Mittel in kosmetischen und in pharmazeutischen Zubereitungen einsetzbar.

Die Wirkungen und positiven Aktivitäten der Extrakte aus Pistia stratiotes enthalten folglich eine sehr starke
- anregende (stimulierende), revitalisierende und regenerierende Aktivität auf den Stoffwechsel,
- eine Apoptose inhibierende Aktivität und damit eine anti-ageing Aktivität und
- eine starke Lipolyse Aktivität was ihren Einsatz in schlankmachenden Pflegemitteln begründet.

### 7. Beispielrezepturen kosmetischer Mittel mit Pistia stratiotes Extrakten

Die gemäß Beispiel 1 bis 3 erhaltenen Pistia stratiotes Extrakte wurden in den folgenden erfindungsgemäßen Rezepturen K1 bis K21 sowie 1 bis 25 eingesetzt. Die so hergestellten kosmetischen Mittel zeigten gegenüber den Vergleichsrezepturen V1, V2 und V3 sehr gute hautpflegende Eigenschaften bei gleichzeitig guter Hautverträglichkeit. Darüber hinaus sind die erfindungsgemäßen Mittel stabil gegen oxidative Zersetzung.

**Tabelle 5: Softcreme Rezepturen K1 bis K7**

| (Alle Angaben in Gew.-% bezogen auf das kosmetische Mittel) | | | | | | | | |
|---|---|---|---|---|---|---|---|---|
| INCI Bezeichnung | K1 | K2 | K3 | K4 | K5 | K6 | K7 | V1 |
| Glyceryl Stearate (and) Ceteareth-12/20 (and) Cetearyl Alcohol (and) Cetyl | 8,0 | 8,0 | 8,0 | 8,0 | 8,0 | 8,0 | 8,0 | 8,0 |
| Palmitate | | | | | | | | |
| Cetearyl Alcohol | 2,0 | 2,0 | 2,0 | 2,0 | 2,0 | 2,0 | 2,0 | 2,0 |
| Dicaprylyl Ether | 2,0 | 2,0 | 2,0 | 2,0 | 2,0 | 2,0 | 2,0 | 2,0 |
| Cocoglycerides | 3,0 | 3,0 | 3,0 | 3,0 | 3,0 | 3,0 | 3,0 | 3,0 |
| Cetearyl Isononanoate | 3,0 | 3,0 | 3,0 | 3,0 | 3,0 | 3,0 | 3,0 | 3,0 |
| Glycerin (86 Gew.-%ig) | 3,0 | 3,0 | 3,0 | 3,0 | 3,0 | 3,0 | 3,0 | 3,0 |
| Extrakt nach Beispiel 1 bis 3 | 0,5 | 0,5 | 0,5 | 0,5 | 0,5 | 0,5 | 0,5 | - |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | 0,5 | | |
| Panthenol | | | | | | | 0,5 | |
| Wasser | | | | Ad 100 | | | | |

**Tabelle 6: Nachtcremerezepturen K8 bis K14**

| (Alle Angaben in Gew.-% bezogen auf das kosmetische Mittel) | | | | | | | | |
|---|---|---|---|---|---|---|---|---|
| INCl Bezeichnung | K8 | K9 | K10 | K11 | K12 | K13 | K14 | V2 |
| Polyglyceryl-2 Dipolyhydroxystearate | 4,0 | 4,0 | 4,0 | 4,0 | 4,0 | 4,0 | 4,0 | 5,0 |
| Polyglyceryl-3 Diisostearate | 2,0 | 2,0 | 2,0 | 2,0 | 2,0 | 2,0 | 2,0 | 2,0 |
| Cera Alba | 2,0 | 2,0 | 2,0 | 2,0 | 2,0 | 2,0 | 2,0 | 2,0 |
| Zinc Stearate | 2,0 | 2,0 | 2,0 | 2,0 | 2,0 | 2,0 | 2,0 | 2,0 |
| Cocoglycerides | 3,0 | 3,0 | 3,0 | 3,0 | 3,0 | 3,0 | 3,0 | 3,0 |
| Cetaeryl Isononanoate | 8,0 | 8,0 | 8,0 | 8,0 | 8,0 | 8,0 | 8,0 | 8,0 |
| Dicaprylyl Ether | 5,0 | 5,0 | 5,0 | 5,0 | 5,0 | 5,0 | 5,0 | 5,0 |
| Magnesiumsulfate | 1,0 | 1,0 | 1,0 | 1,0 | 1,0 | 1,0 | 1,0 | 1,0 |
| Glycerin (86 Gew.-%ig) | 5,0 | 5,0 | 5,0 | 5,0 | 5,0 | 5,0 | 5,0 | 5,0 |
| Extrakt nach Beispiel 1 bis 3 | 0,5 | 0,5 | 0,5 | 0,5 | 0,5 | 0,5 | 0,5 | - |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | 0,5 | | |
| Panthenol | | | | | | | 0,5 | |
| Wasser | | | | Ad 100 | | | | |

**Tabelle 7: W/O Bodylotion Rezepturen K15 bis K21**

| (Alle Angaben in Gew.-% bezogen auf das kosmetische Mittel) | | | | | | | | |
|---|---|---|---|---|---|---|---|---|
| INCI-Bezeichnung | K15 | K16 | K17 | K18 | K19 | K20 | K21 | V3 |
| PEG-7 Hydrogenated Castor Oil | 7,0 | 7,0 | 7,0 | 7,0 | 7,0 | 7,0 | 7,0 | 7,0 |
| Decyl Oleate | 7,0 | 7,0 | 7,0 | 7,0 | 7,0 | 7,0 | 7,0 | 7,0 |
| Cetearyl Isononanoate | 7,0 | 7,0 | 7,0 | 7,0 | 7,0 | 7,0 | 7,0 | 7,0 |
| Glycerin (86 Gew.-%ig) | 5,0 | 5,0 | 5,0 | 5,0 | 5,0 | 5,0 | 5,0 | 5,0 |
| MgSO₄·7 H₂O | 1,0 | 1,0 | 1,0 | 1,0 | 1,0 | 1,0 | 1,0 | 1,0 |
| Extrakt nach Beispiel 1 bis 3 | 1,5 | 1,5 | 1,5 | 1,5 | 1,5 | 1,5 | 1,5 | |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹) | | | | | | 0,5 | | |
| Panthenol | | | | | | | 0,5 | |
| Wasser | | | | Ad 100 | | | | |

| | | | | | | | | |
|---|---|---|---|---|---|---|---|---|
| ¹⁾ Desoxyribonucleinsäure: Molekuargewicht ca. 70000, Reinheit (bestimmt durch spektro-photometrische Messung der Absorption bei 260 nm sowie 280 nm): mindestens 1,7. | | | | | | | | |

**Tabelle 8: Rezepturen**

| **Kosmetische Zubereitungen Conditioner** (Alle Angaben in Gew.-% bezogen auf das kosmetische Mittel, Wasser, Konservierungsmittel addieren sich zu 100 Gew.-%) | | | | | | |
|---|---|---|---|---|---|---|
| **Zusammensetzung (INCl)** | **1 Gew.- %** | **2 Gew.- %** | **3 Gew.- %** | **4 Gew.- %** | **5 Gew.- %** | **6 Gew.-%** |
| **Dehyquart® A** Cetrimonium Chloride | 4,0 | 4,0 | | | 3,0 | |
| **Dehyquart L® 80** Dicocoylmethylethoxymonium Methosulfate (and) Propylenglycol | | | 1,2 | 1,2 | | 1,0 |
| **Eumulgin® B2** Ceteareth-20 | 0,8 | | - | 0,8 | - | 1,0 |
| **Eumulgin® VL 75** Lauryl Glucoside (and) Polyglyceryl-2 Polyhydroxystearate (and) Glycerin | - | 2,0 | 2,0 | - | 0.8 | - |
| **Lanette® O** Cetearyl Alcohol | 3,0 | 3,0 | 3,0 | 3,0 | 3,0 | 3,0 |
| **Cutina® GMS** Glyceryl Stearate | - | 0,5 | - | 0,5 | - | 1,0 |
| **Lamesoft® PO 65** Coco-Glucosid (and) Glyceryl Oleate | | - | 3,0 | - | - | 3,0 |
| **Cetiol® J 600** Oleyl Erucate | - | 0,5 | - | 1,0 | - | 1,0 |
| **Eutanol® G** Octyldodecanol | - | - | 1,0 | - | - | 1,0 |
| **Nutrilan® Keratin W** Hydrolyzed Keratin | 5,0 | - | - | 2,0 | - | - |
| **Generol® 122 N** Soja Sterol | - | - | - | - | 1,0 | 1,0 |
| **Extrakt aus Pistia stratiotes** | 1,0 | 1,0 | 1,0 | 1,0 | 1,0 | 1,0 |
| **Copherol® 1250** Tocopheryl Acetate | - | - | 0,1 | 0,1 | - | - |

| | | | | | | |
|---|---|---|---|---|---|---|
| (1-4) Haarspülung, (5-6) Haarkur | | | | | | |

**Tabelle 8**

| **Rezepturen für Conditioner II** **Kosmetische Zubereitungen Conditioner** (Alle Angaben in Gew.-% bezogen auf das kosmetische Mittel Wasser, Konservierungsmittel addieren sich zu 100 Gew.-%) | | | | |
|---|---|---|---|---|
| **Zusammensetzung (INCl)** | **7 Gew.- %** | **8 Gew.- %** | **9 Gew.- %** | **10 Gew.-%** |
| **Texapon® NSO** Sodium Laureth Sulfate | 38,0 | 38,0 | 25,0 | - |
| **Texapon® SB 3** Disodium Laureth Sulfosuccinate | - | - | 10,0 | - |
| **Plantacare® 818** Coco Glucosides | 7,0 | 7,0 | 6,0 | - |
| **Plantacare® PS 10** Sodium Laureth Sulfate (and) Coco Glucosides | - | - | - | 20,0 |
| **Dehyton® PK 45** Cocamidopropyl Betaine | - | - | 10,0 | |
| **Lamesoft® PO 65** Coco-Glucosid (and) Glyceryl Oleate | 3,0 | | | 4,0 |
| **Lamesoft® LMG** Glyceryl Laurate (and) Potassium Cocoyl Hydrolyzed Collagen | - | 5,0 | - | |
| **Euperlan® PK 3000 AM** Glycol Distearate (and) Laureth-4 (and) Cocamidopropyl Betaine | - | 3,0 | 5,0 | 5,0 |
| **Extrakt aus Pistia stratiotes** | 1,0 | 1,0 | 1,0 | 1,0 |
| **Arlypon® F** Laureth-2 | 3,0 | 3,0 | 1,0 | - |
| **Sodium Chloride** | - | 1,5 | - | 1,5 |

| | | | | |
|---|---|---|---|---|
| (7-8) Duschbad, (9) Duschgel, (10) Waschlotion | | | | |

**Tabelle 8**

| **Kosmetische Zubereitungen Duschbad "Two in One" (Wasser, Konservierungsmittel ad 100 Gew.-%) - Fortsetzung** | | | | |
|---|---|---|---|---|
| **Zusammensetzung (INCl)** | **11** | **12** | **13** | **14** |
| **Texapon NSO** Sodium Laureth Sulfate | 30,0 | 25,0 | | 25,0 |
| **Plantacare 818** Coco Glucosides | | | | 8,0 |
| **Plantacare 2000** Decyl Glucoside | | 8,0 | | |
| **Plantacare PS 10** Sodium Laureth Sulfate (and) Coco Glucosides | | | 20,0 | |
| **Dehyton PK 45** Cocamidopropyl Betaine | | 10,0 | 10,0 | |
| **Lamesoft PO 65** Coco-Glucosid (and) Glyceryl Oleate | 5,0 | | | |
| **Lamesoft LMG** Glyceryl Laurate (and) Potassium Cocoyl Hydrolyzed Collagen | | 5,0 | 5,0 | |
| **Gluadin WQ** Laurdimonium Hydroxapropyll Hydrolyzed Wheat Protein | 3,0 | | | |
| **Gluadin WK** Sodium Cocoyl Hydrolyzed Wheat Protein | | | | |
| **Euperlan PK 3000 AM** Glycol Distearate (and) Laureth-4 (and) Cocamidopropyl Betaine | 5,0 | 3,0 | 4,0 | - |
| **Panthenol** | 0,5 | - | - | 0,5 |
| **Extrakt aus Pistia stratiotes** | 1,0 | 1,0 | 1,0 | 1,0 |
| **Arlypon F** Laureth-2 | 2,6 | 1,6 | - | 1,0 |
| **Sodium Chloride** | - | - | - | - |

**Tabelle 8**

| **Kosmetische Zubereitungen Shampoo** (Alle Angaben in Gew.-% bezogen auf das kosmetische Mittel, Wasser, Konservierungsmittel addieren sich zu 100 Gew.-%) | | | | | | |
|---|---|---|---|---|---|---|
| **Zusammensetzung (INCl)** | **15** | **16** | **17** | **18** | **19** | **20** |
| **Texapon® NSO** Sodium Laureth Sulfate | 30,0 | | | 30,0 | 25,0 | |
| **Texapon® K 14 S** Sodium Myreth Sulfate | | 30,0 | | | | 30,0 |
| **Texapon® SB 3** Disodium Laureth Sulfosuccinate | | 10,0 | | | | |
| **Plantacare® 818** Coco Glucosides | 4,0 | | | | | |
| **Plantacare® 2000** Decyl Glucoside | | 4,0 | | | | |
| **Plantacare® PS 10** Sodium Laureth Sulfate (and) Coco Glucosides | | | 20,0 | | | |
| **Dehyton® PK 45** Cocamidopropyl Betaine | 5,0 | | | 10,0 | | 10,0 |
| **Gluadin® WK** Sodium Cocoyl Hydrolyzed Wheat Protein | | | | | 8,0 | |
| **Lamesoft® PO 65** Coco-Glucosid (and) Glyceryl Oleate | - | - | - | - | 2,0 | 2,0 |
| **Nutrilan® Keratin W** Hydrolyzed Keratin | 5,0 | - | - | - | | - |
| **Gluadin® W 40** Hydrolyzed Wheat Protein | - | 2,0 | - | 2,0 | - | - |
| **Euperlan® PK 3000 AM** Glycol Distearate (and) Laureth-4 (and) Cocamidopropyl Betaine | - | - | - | 3,0 | 3,0 | - |
| **Panthenol** | - | - | - | - | - | 0,2 |
| **Extrakte aus Pistia stratiotes** | 1,0 | 1,0 | 1,0 | 1,0 | 1,0 | 1,0 |
| **Arlypon® F** Laureth-2 | 1,5 | - | - | - | - | - |
| **Sodium Chloride** | - | 1,6 | 2,0 | 2,2 | - | 3,0 |

**Tabelle 8**

| **Kosmetische Zubereitungen Schaumbad** (Alle Angaben in Gew.-% bezogen auf das kosmetische Mittel, Wasser, Konservierungsmittel addieren sich zu 100 Gew.-%) | | | | | |
|---|---|---|---|---|---|
| **Zusammensetzung** Bezeichnung nach INCl | **21** | **22** | **23** | **24** | **25** |
| **Texapon® NSO** Sodium Laureth Sulfate | - | 30,0 | 30,0 | - | 25,0 |
| **Plantacare® 818** Coco Glucosides | - | 10,0 | - | - | 20,0 |
| **Plantacare® PS 10** Sodium Laureth Sulfate (and) Coco Glucosides | 22,0 | - | 5,0 | 22,0 | - |
| **Dehyton® PK 45** Cocamidopropyl Betaine | 15,0 | 10,0 | 15,0 | 15,0 | 20,0 |
| **Monomuls® 90-O 18** Glyceryl Oleate | 0,5 | | | | |
| **Lamesoft® PO 65** Coco-Glucosid (and) Glyceryl Oleate | | 3,0 | | 3,0 | |
| **Cetiol® HE** PEG-7 Glyceryl Cocoate | | | 2,0 | | 2,0 |
| **Nutrilan® I-50** Hydrolyzed Collagen | 5,0 | | | | |
| **Gluadin® W 40** Hydrolyzed Wheat Gluten | | 5,0 | | 5,0 | |
| **Gluadin® WK** Sodium Cocoyl Hydrolyzed Wheat Protein | | | | 7,0 | |
| **Euperlan® PK 3000 AM** Glycol Distearate (and) Laureth-4 (and) Cocamidopropyl Betaine | 5,0 | - | - | 5,0 | - |
| **Arlypon® F** Laureth-2 | | | 1,0 | | |
| **Sodium Chloride** | 1,0 | | 1,0 | | 2,0 |
| **Extrakte aus Pistia stratiotes** | 1,0 | 1,0 | 1,0 | 1,0 | 1,0 |

Alle in der Tabelle 8 aufgeführten und verwendeten Substanzen mit registriertem Warenzeichen ® sind Marken und Produkte der COGNIS Gruppe.

## Patentansprüche

1. Kosmetische Verwendung von Extrakten der Pflanze Pistia stratiotes zur Haut- oder Haarpflege.

2. Verwendung nach Anspruch 1 zur revitalisierenden und reaktivierenden Haut- oder Haarpflege.

3. Kosmetische Verwendung von Extrakten der Pflanze Pistia stratiotes in Pflegemitteln mit schlankmachenden und Anti-cellulitis Eigenschaften für die Haut.

4. Kosmetische Verwendung von Extrakten der Pflanze Pistia stratiotes in Pflegemitteln zur vorbeugenden oder heilenden Behandlung von Alterserscheinungen der Haut.

5. Verwendung nach Anspruch 4, **dadurch gekennzeichnet, dass** die durch UV-Strahlung induzierten Alterserscheinungen der Haut behandelt werden.

6. Verwendung nach Anspruch 4, **dadurch gekennzeichnet, dass** die durch Mangel an Wachstumsfaktoren induzierte Apoptose und damit induzierten Alterserscheinungen der Haut behandelt werden.

## Claims

1. Cosmetic use of extracts of the plant Pistia stratiotes for skin and/or hair care

2. Use according to claim 1 for revitalizing and reactivating skin or hair care.

3. Cosmetic use of extracts of the plant Pistia stratiotes in care compositions with slimming and anti-cellulites properties for the skin.

4. Cosmetic use of extracts of the plant Pistia stratiotes in care compositions for the preventive or healing treatment of signs of skin ageing.

5. Use according to claim 4, **characterized in that** UV-induced skin ageing is treated.

6. Use according to claim 4, **characterized in that** apoptosis induced by a deficiency of growth factors and correspondingly induced signs of skin ageing are treated.

## Revendications

1. Utilisation cosmétique d'extraits de la plante Pistia stratiotes pour le soin de la peau ou des cheveux.

2. Utilisation selon la revendication 1 pour le soin revitalisant et réactivant de la peau ou des cheveux.

3. Utilisation cosmétique d'extraits de la plante Pistia stratiotes dans des agents ou produits de soin présentant des propriétés amincissantes et des propriétés anti-cellulite pour la peau.

4. Utilisation cosmétique d'extraits de la plante Pistia stratiotes dans des agents ou produits de soin pour un traitement préventif ou curatif des manifestations de vieillissement cutané.

5. Utilisation selon la revendication 4, **caractérisée en ce que** l'on traite les manifestations de vieillissement cutané induites par le rayonnement ultraviolet.

6. Utilisation selon la revendication 4, **caractérisée en ce que** l'on traite l'apoptose induite par une carence en facteurs de croissance et les manifestations de vieillissement cutané ainsi induites.
